# EUROPÄISCHE PATENTSCHRIFT

(11) **EP 4 175 810 B1**
(45) Veröffentlichungstag und Bekanntmachung des Hinweises auf die Patenterteilung: **22.10.2025**
(21) Anmeldenummer: 21739992.2
(22) Anmeldetag: 30.06.2021
(51) Int. Cl.: B29C 45/16, A61F 2/00

(54) **BANDFÖRMIGE MEDIZINISCHE EINRICHTUNG ZUM VERENGEN EINES KÖRPERKANALS UND VERFAHREN ZU DEREN HERSTELLUNG**
STRIP-LIKE MEDICAL DEVICE FOR NARROWING A BODILY CONDUIT AND PROCESS FOR MAKING IT
DISPOSITIF MÉDICAL EN FORME DE BANDE SERVANT À RÉTRÉCIR UN CONDUIT CORPOREL ET SON PROCÉDÉ DE FABRICATION

(30) Priorität: 06.07.2020 AT 1502020
(43) Veröffentlichungstag der Anmeldung: 10.05.2023
(73) Patentinhaber: A.M.I. Agency for Medical Innovations GmbH, 6800 Feldkirch (AT)
(72) Erfinder: HOHLRIEDER, Martin, 6840 Götzis (AT); ERHARD, Martin, 6780 Silbertal (AT)
(74) Vertreter: Torggler & Hofmann Patentanwälte - Rankweil
(86) Internationale Anmeldenummer: PCT/EP2021/067988
(87) Internationale Veröffentlichungsnummer: WO 2022/008313

(56) Entgegenhaltungen:
- US-A- 5 133 754
- US-A1- 2005 119 672

## Beschreibung

Die Erfindung bezieht sich auf eine bandförmige medizinische Einrichtung zum Verengen eines Körperkanals eines Patienten, wobei die Einrichtung um das den Körperkanal umgebende Körpergewebe legbar ist, und wobei die Einrichtung umfasst: einen ersten Abschnitt aus einer ersten Silikonzusammensetzung, und einen zweiten Abschnitt aus einer zweiten Silikonzusammensetzung, die gegenüber der ersten Silikonzusammensetzung eine geringere Shore-A Härte aufweist, und einen Verbindungsabschnitt, der den ersten Abschnitt mit dem zweiten Abschnitt stoffschlüssig verbindet, wobei der erste Abschnitt ein erstes Verschlussteil und der zweite Abschnitt ein zweites Verschlussteil aufweist, mittels denen die Einrichtung zu einem Ring schließbar ist, der eine Durchtrittsöffnung für das den Körperkanal umgebende Körpergewebe umschließt.

Bandförmig ausgebildete medizinische Einrichtungen der genannten Art werden im menschlichen Körper beispielsweise zur Unterstützung geschwächter natürlicher Muskeln angewendet. Ein Einsatzbeispiel derartiger Einrichtungen sind z.B. zu einem Ring schließbare bandförmige Einrichtungen zum Verengen und/oder Absperren eines Hohlorgans, z.B. des Mastdarms im Bereich des Anus.

Je nach Einsatzzweck weisen die voneinander verschiedenen Silikonzusammensetzungen, aus denen der erste Abschnitt und der zweite Abschnitt der eingangs genannten medizinischen Einrichtung bestehen, unterschiedliche mechanische Eigenschaften auf. Beispielsweise ist es beim Einsatz der medizinischen Einrichtung zur Unterstützung oder zum Ersatz von geschwächten natürlichen Muskeln im Bereich des Anus nötig, dass einerseits eine zuverlässige Funktion zur Behandlung von Stuhlinkontinenz gewährleistet und andererseits ein angenehmes Tragegefühl für den Patienten bereitgestellt wird. In diesem Einsatzgebiet ist beispielsweise die Bereitstellung einer relativ hohen Elastizität der Einrichtung und gleichzeitig ein sicherer Verschluss gefordert.

Im Medizinbereich ist es bekannt, einen ersten Abschnitt einer medizinischen Einrichtung aus einer ausgehärteten ersten Silikonzusammensetzung mit einem zweiten Abschnitt aus einer ausgehärteten zweiten Silikonzusammensetzung, die gegenüber der ersten Silikonzusammensetzung eine geringere Shore-A Härte aufweist, miteinander zu verkleben. D.h., der Verbindungsabschnitt wird in diesen Fällen von einer dünnen Schicht eines geeigneten Klebstoffs gebildet, der den ersten Abschnitt mit dem zweiten Abschnitt stoffschlüssig verbindet.

Es wurde festgestellt, dass die Zuverlässigkeit der Verklebung der beiden Abschnitte oft nur bei geringen auftretenden Kräften gewährleistet ist und es bei größeren auftretenden Kräften zu einer zumindest bereichsweisen Zerstörung der Verklebung kommt. Insbesondere für den zuvor erläuterten Anwendungsfall im Bereich des Anus ist eine derartige Verklebung aufgrund der auftretenden Kräfte nur unter großem Aufwand einsetzbar. Beispielsweise müssen große, in Längsrichtung des Bandes verlaufende Überlappungsbereiche der mit Klebstoff zu verbindenden Abschnitte vorgesehen werden, welche eine die Funktion und den Tragekomfort beeinträchtigende Versteifung der Einrichtung zur Folge hat.

Aus der US 2005/119672 A1 geht ein Magenband hervor, welches eine mit Flüssigkeit befüllbare Hohlkammer zur Verkleinerung einer Durchtrittsöffnung des zu einem Ring geschlossenen Magenbandes aufweist. Das Magenband ist aus Silikon gefertigt, wobei zwei Silikonzusammensetzungen zum Einsatz kommen. Auf der Seite der Hohlkammer, welche von der Durchtrittsöffnung entfernt liegt, ist eine Rückenverstärkung aus einer härteren Silikonzusammensetzung vorhanden. Aus dieser härteren Silikonzusammensetzung besteht im Weiteren ein ringförmiger Bereich zum Verstärken eines Verschlussteils des Magenbandes. Die härteren Silikonzusammensetzungen werden in einem ersten Verfahrensschritt hergestellt und in der Folge mit einer weicheren Silikonzusammensetzung umspritzt.

Aus der US 5133 754 A gehen Implantate zur Imitation der Haptik von Knorpel- oder Knochenstrukturen, z.B. im Bereich der Nase oder des Kinns hervor. Diese Implantate besitzen hierfür zwei unterschiedliche Silikonzusammensetzungen, um unterschiedliche Haptiken auszubilden, wobei die beiden Silikonzusammensetzungen gleichzeitig in eine Form eingespritzt werden und sich hierbei auch verbinden.

Aufgabe der Erfindung ist es, eine vorteilhafte Einrichtung der eingangs genannten Art bereitzustellen, welche zuverlässig eingesetzt werden kann und für den Patienten angenehm zu tragen ist.

Bei der Einrichtung gemäß der Erfindung ist vorgesehen, dass der Verbindungsabschnitt aus einer Mischung der ersten Silikonzusammensetzung und der zweiten Silikonzusammensetzung besteht. Der Verbindungsabschnitt, der den ersten Abschnitt und den zweiten Abschnitt der Einrichtung miteinander stoffschlüssig verbindet, weist somit ausschließlich eine Mischung der ersten Silikonzusammensetzung und der zweiten Silikonzusammensetzung auf. D.h., dass auf zusätzliche Klebstoffe oder andere Verbindungsmittel zwischen dem ersten Abschnitt und dem zweiten Abschnitt verzichtet wird.

Überraschenderweise wurde festgestellt, dass unterschiedliche Silikonzusammensetzungen, die sich im flüssigen Zustand, z.B. in einer Form, berühren, beim Aushärten einen Verbindungsabschnitt bilden, der aus beiden Silikonzusammensetzungen besteht. Die Moleküle der unterschiedlichen Silikonzusammensetzungen vernetzen und/oder verketten sich im Verbindungsabschnitt auf molekularer Ebene direkt miteinander. Dadurch kann die Einrichtung großen Kräften ausgesetzt werden, ohne im Bereich des Verbindungsabschnitts zu reißen.

Die Verteilung der Anteile der ersten Silikonzusammensetzung und der zweiten Silikonzusammensetzung im Verbindungsabschnitt ist dabei nicht zwingend homogen. Insbesondere kann die Konzentration der jeweiligen Silikonzusammensetzung zum jeweils an den Verbindungsabschnitt anschließenden Abschnitt, der ausschließlich aus dieser Silikonzusammensetzung besteht, hin zunehmen und die Konzentration der anderen Silikonzusammensetzung entsprechend abnehmen. Der Verbindungsabschnitt könnte somit auch als Übergangsabschnitt bezeichnet werden.

Durch das Vorsehen eines ersten Abschnitts aus einer ersten Silikonzusammensetzung und eines zweiten Abschnitts aus einer zweiten Silikonzusammensetzung können unterschiedliche Materialeigenschaften für den jeweiligen Zweck bzw. die gewünschte Funktion der Einrichtung vorgesehen werden. Der Verbindungsabschnitt, der aus einer Mischung der ersten Silikonzusammensetzung und der zweiten Silikonzusammensetzung besteht, ermöglicht eine einfache Ausbildung der medizinischen Einrichtung ohne eine für den Patienten störende Materialanhäufung im Bereich des Verbindungsabschnitts, da beispielsweise bei der Ausbildung als Band auf eine auf die Längsrichtung des Bandes bezogene Überlappung der Abschnitte verzichtet werden kann.

Die Shore-A Härte von Silikonzusammensetzungen wird im, insbesondere vollständig, ausgehärteten Zustand der Silikonzusammensetzungen gemessen. Dieser Zustand könnte auch als Endhärte der jeweiligen Silikonzusammensetzung bezeichnet werden. Die Shore-A Härte kann beispielsweise nach der Norm DIN ISO 7619-1:2012-02 ermittelt werden.

Bevorzugt weist die erste Silikonzusammensetzung eine Shore-A Härte in einem Bereich von 30 bis 90, vorzugsweise in einem Bereich von 40 bis 80, besonders bevorzugt in einem Bereich von 60 bis 75, auf.

Die zweite Silikonzusammensetzung weist gegenüber der ersten Silikonzusammensetzung günstigerweise eine zumindest um den Wert 15, besonders bevorzugt um den Wert 30, geringere Shore-A Härte auf.

Die Shore-A Härte der zweiten Silikonzusammensetzung liegt günstigerweise in einem Bereich von 2 bis 15, vorzugsweise in einem Bereich von 3 bis 10, besonders bevorzugt in einem Bereich von 4 bis 7.

In einer möglichen Ausführungsform weist die Einrichtung einen dritten Abschnitt aus einer dritten Silikonzusammensetzung und einen Zusatz-Verbindungsabschnitt auf, wobei der Zusatz-Verbindungsabschnitt den dritten Abschnitt mit dem zweiten Abschnitt stoffschlüssig verbindet und der Zusatz-Verbindungsabschnitt aus einer Mischung der dritten Silikonzusammensetzung und der zweiten Silikonzusammensetzung besteht. Die dritte Silikonzusammensetzung kann allenfalls eine größere Shore-A Härte als der zweite Abschnitt aufweisen, insbesondere kann die dritte Silikonzusammensetzung identisch zur ersten Silikonzusammensetzung sein.

Bevorzugt weist die dritte Silikonzusammensetzung eine Shore-A Härte in einem Bereich von 30 bis 70, besonders bevorzugt in einem Bereich von 40 bis 60, auf.

Mögliche Anwendungsgebiete der um das den Körperkanal umgebende Körpergewebe legbaren bandförmigen medizinischen Einrichtung sind beispielsweise künstliche Sphinkter zur Verengung des Mastdarms oder des Harnleiters. Als weiteres mögliches Anwendungsgebiet könnte die Einrichtung als künstlicher unterer Ösophagussphinkter im Bereich des Mageneingangs um die Speiseröhre gelegt werden. Die zu einem Ring geschlossene Einrichtung umschließt die Durchtrittsöffnung für das den Körperkanal umgebende Körpergewebe, z.B. geschwächte natürliche Muskeln.

Der erste und zweite Abschnitt und der gegebenenfalls vorgesehene dritte Abschnitt sind, in einer Längserstreckungsrichtung der bandförmigen Einrichtung gesehen, hintereinander angeordnet.

Es kann vorgesehen sein, dass das erste Verschlussteil eine Einstecköffnung aufweist. Das erste Verschlussteil mit Einstecköffnung könnte auch als Öse bezeichnet werden.

Im Weiteren kann vorgesehen sein, dass das zweite Verschlussteil in die Einstecköffnung einführbar ist und gegenüber dem ersten Verschlussteil im geschlossenen Zustand verrastbar ist. Das am ersten Abschnitt angeordnete erste Verschlussteil ermöglicht durch die Ausbildung aus der härteren ersten Silikonzusammensetzung ein zuverlässiges Schließen der zu einem Ring schließbaren bandförmigen Einrichtung.

Das zweite Verschlussteil kann in einer möglichen Ausführungsform zumindest zwei Rastnasen aufweisen, die zur Einstellung der Größe der Durchtrittsöffnung wahlweise mit dem ersten Verschlussteil verrastbar sind. Dies ermöglicht die Anpassung der Größe der Durchtrittsöffnung an den individuellen Patienten bzw. die Abmessungen des zu umschließenden Körperkanals.

Günstigerweise ist vorgesehen, dass die Einrichtung einstückig ausgebildet ist. Dies ermöglicht einen einfachen Aufbau der Einrichtung.

Bei den Silikonzusammensetzungen handelt es sich günstigerweise um sogenanntes medizinisches Silikon, welches körperverträglich ist. Die Silikonzusammensetzungen können als Einkomponenten- oder Zweikomponentensilikon vorliegen.

Die Erfindung bezieht sich im Weiteren auf ein Verfahren zur Herstellung einer medizinischen Einrichtung, welches vorsieht, dass, die erste Silikonzusammensetzung in einem flüssigen Zustand über eine im Bereich des auszubildenden ersten Abschnitts mündende erste Einfüllöffnung in einen Aufnahmeraum einer Form eingefüllt wird und die zweite Silikonzusammensetzung in einem flüssigen Zustand über eine im Bereich des auszubildenden zweiten Abschnitts mündende zweite Einfüllöffnung in den Aufnahmeraum der Form eingefüllt wird, wobei sich die erste Silikonzusammensetzung und die zweite Silikonzusammensetzung im flüssigen Zustand im Aufnahmeraum berühren und sich über den Bereich des auszubildenden Verbindungsabschnitts vermischen. Zur Bildung der medizinischen Einrichtung ist vorgesehen, dass die erste Silikonzusammensetzung im ersten Abschnitt der Einrichtung und die zweite Silikonzusammensetzung im zweiten Abschnitt der Einrichtung und die Mischung der ersten Silikonzusammensetzung und der zweiten Silikonzusammensetzung im Verbindungsabschnitt, insbesondere gemeinsam, ausgehärtet werden.

Weist die medizinische Einrichtung noch einen weiteren Abschnitt auf, z.B. den oben erwähnten dritten Abschnitt aus einer dritten Silikonzusammensetzung, so ist entsprechend die dritte Silikonzusammensetzung in einem flüssigen Zustand über eine im Bereich des auszubildenden dritten Abschnitts mündende dritte Einfüllöffnung in den Aufnahmeraum der Form einzufüllen. Die zweite Silikonzusammensetzung und die dritte Silikonzusammensetzung berühren sich im flüssigen Zustand im Aufnahmeraum und vermischen sich über den Bereich des auszubildenden Zusatz- Verbindungsabschnitts. Zur Bildung der medizinischen Einrichtung mit drei Abschnitten ist dann entsprechend zusätzlich vorzusehen, dass die dritte Silikonzusammensetzung im dritten Abschnitt und die Mischung der zweiten Silikonzusammensetzung und der dritten Silikonzusammensetzung im Zusatz-Verbindungsabschnitt ausgehärtet werden.

Das Aushärten der Silikonzusammensetzungen kann in einem Ofen erfolgen.

Optional ist es denkbar, dass im Bereich des jeweils zu bildenden Verbindungsabschnitts ein verschiebbar gelagerter Trennschieber angeordnet ist. Der Trennschieber unterteilt den Aufnahmeraum jeweils in Teil-Aufnahmeräume und kann nach dem Einfüllen der Silikonzusammensetzungen aus dem Aufnahmeraum der Form entfernt werden, wobei sich die Silikonzusammensetzungen nach dem Entfernen des Trennschiebers berühren und sich im Verbindungsabschnitt vermischen.

Die Länge des Verbindungsabschnitts und/oder des Zusatz-Verbindungsabschnitts der Einrichtung beträgt, in Längsrichtung der bandförmigen Einrichtung gemessen, günstigerweise mindestens 1mm, vorzugsweise mindestens 3mm. Bevorzugt erstreckt sich die Länge des Verbindungsabschnitts und/oder des Zusatz-Verbindungsabschnitts in Längsrichtung der bandförmigen Einrichtung über weniger als 1,5cm, besonders bevorzugt über weniger als 1 cm.

Weitere Merkmale und Einzelheiten der Erfindung werden anhand des in den Figuren gezeigten Ausführungsbeispiels einer erfindungsgemäßen medizinischen Einrichtung und den in den Figuren gezeigten Beispielen erfindungsgemäßer Verfahrensschritte zur Herstellung der Einrichtung erläutert. Es zeigen:
- Fig. 1: einen Schnitt durch eine Form zur Herstellung einer erfindungsgemäßen medizinischen Einrichtung, wobei eine erste Silikonzusammensetzung in die Form eingefüllt ist;
- Fig. 2: eine Darstellung analog zu Fig. 1, bei der bereits ein Teil einer zweiten Silikonzusammensetzung in die Form eingefüllt ist;
- Fig. 3: eine Darstellung analog zu Fig. 1, bei der die erste und zweite Silikonzusammensetzung vollständig eingefüllt sind;
- Fig. 4: die ausgehärtete und entformte medizinische Einrichtung gemäß Fig. 3;
- Fig. 5 und 6: eine alternative Ausführungsform einer Form zur Herstellung einer erfindungsgemäßen Einrichtung mit einem Trennschieber;
- Fig. 7: eine erfindungsgemäße medizinische Einrichtung zur Ausbildung eines artifiziellen Sphinkters in einer isometrischen Darstellung;
- Fig. 8: eine Vorderansicht der Einrichtung gemäß Fig. 7;
- Fig. 9: eine Draufsicht auf die Einrichtung gemäß Fig. 7;
- Fig. 10: die Einrichtung gemäß Fig. 7 in einem geschlossenen Zustand;
- Fig. 11: die Einrichtung gemäß Fig. 10 im implantierten Zustand;
- Fig. 12: eine schematische Darstellung des implantierten Zustands der Einrichtung im menschlichen Körper;
- Fig. 13: eine Form zur Herstellung der medizinischen Einrichtung gemäß der Fig. 7-12, und
- Fig. 14: den Schnitt A-A der Fig. 13.

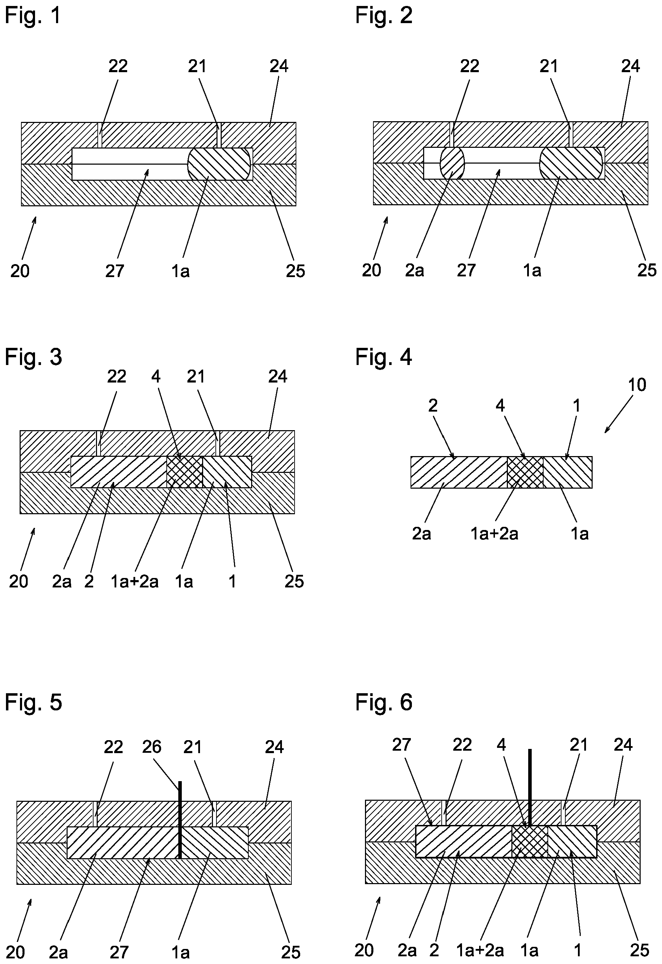

In den folgenden Erläuterungen wird zuerst auf ein erfindungsgemäßes Verfahren zur Herstellung einer medizinischen Einrichtung gemäß der Erfindung eingegangen, die in Fig. 4 schraffiert und stark vereinfacht dargestellt ist. Die unterschiedlichen Silikonzusammensetzungen sind in den Fig. 1 bis 4 unterschiedlich schraffiert dargestellt. Aus Gründen der Übersichtlichkeit wurde in den Fig. 1 bis 3 auf die Darstellung von Dosierpumpen zur Einfüllung von Silikonzusammensetzungen etc. verzichtet. Derartige Einrichtungen sind hinlänglich bekannt.

In Fig. 1 ist eine Form 20 zur Herstellung einer medizinischen Einrichtung 10 dargestellt, die eine untere Formhälfte 25 und eine von der unteren Formhälfte 25 abnehmbare obere Formhälfte 24 aufweist. Zwischen den Formhälften 24, 25 ist ein Aufnahmeraum 27 angeordnet.

Im Ausführungsbeispiel sind in der oberen Formhälfte 24 eine erste Einfüllöffnung 21 und eine zweite Einfüllöffnung 22 vorgesehen, die in den Aufnahmeraum 27 münden. Die erste Einfüllöffnung 21 ist in einem Bereich eines auszubildenden ersten Abschnitts 1 der medizinischen Einrichtung 10 und die zweite Einfüllöffnung 22 in einem Bereich eines auszubildenden zweiten Abschnitts 2 der medizinischen Einrichtung 10 angeordnet.

In einem ersten Schritt wird eine Silikonzusammensetzung 1a in einem flüssigen Zustand über die erste Einfüllöffnung 21 in den Aufnahmeraum 27 der Form 20 eingefüllt, vgl. Fig. 1. Das Volumen der in den Aufnahmeraum 27 eingefüllten ersten Silikonzusammensetzung 1a ist mit einer entsprechenden Dosierpumpe vorbestimmbar. In Fig. 1 ist ersichtlich, dass die erste Silikonzusammensetzung 1a nur einen Teil des Aufnahmeraums 27 ausfüllt.

Im Weiteren wird nun eine flüssige zweite Silikonzusammensetzung 2a über die zweite Einfüllöffnung 22 in den Aufnahmeraum 27 eingefüllt, vgl. Fig. 2. Die zweite Silikonzusammensetzung 2a unterscheidet sich von der ersten Silikonzusammensetzung 1a hinsichtlich der mechanischen Eigenschaften, die die zweite Silikonzusammensetzung 2a im ausgehärteten bzw. verfestigten Zustand einnimmt. Insbesondere weist die zweite Silikonzusammensetzung 2a eine geringere Shore-A Härte als die erste Silikonzusammensetzung 1a auf. Während des Einfüllens der zweiten Silikonzusammensetzung 2a wird die Einfüllöffnung 21 verschlossen oder die Dosierpumpe zur Zufuhr der ersten Silikonzusammensetzung 1a blockiert. Dadurch wird verhindert, dass die erste Silikonzusammensetzung 1a beim Einbringen der zweiten Silikonzusammensetzung 2a über die erste Einfüllöffnung 21 aus dem Aufnahmeraum 27 verdrängt wird.

In Fig. 3 ist ein Zustand dargestellt, bei dem die zweite Silikonzusammensetzung 2a zur Gänze in den Aufnahmeraum 27 eingefüllt ist und den Aufnahmeraum 27 zusammen mit der ersten Silikonzusammensetzung 1a vollständig ausfüllt.

Die erste Silikonzusammensetzung la und die zweite Silikonzusammensetzung 2a berühren sich im flüssigen Zustand im Aufnahmeraum 27 und vermischen sich in einem Bereich eines auszubildenden Verbindungsabschnitts 4, vgl. Fig. 3. In den Figuren ist die Mischung der ersten Silikonzusammensetzung 1a mit der zweiten Silikonzusammensetzung 2a mit 1a+2a bezeichnet.

Luft, die sich vor dem Einfüllen im Aufnahmeraum 27 befindet, kann während des Einfüllens der Silikonzusammensetzungen 1a, 2a über die nicht näher bezeichneten Trennflächen zwischen der oberen Formhälfte 24 und der unteren Formhälfte 25 entweichen.

Zur Bildung der medizinischen Einrichtung 10 werden die erste Silikonzusammensetzung 1a im ersten Abschnitt 1 der Einrichtung 10 und die zweite Silikonzusammensetzung 2a im zweiten Abschnitt 2 der Einrichtung 10 und die Mischung 1a+2a der ersten Silikonzusammensetzung 1a und der zweiten Silikonzusammensetzung 2a im Verbindungsabschnitt 4 gemeinsam ausgehärtet. Das vollständige Aushärten kann durch sogenanntes Tempern bei erhöhter Temperatur, z.B. in einem Vakuumofen, erfolgen, wie dies an sich bekannt ist.

Im Verbindungsabschnitt 4 kommt es beim Aushärten der Silikonzusammensetzungen 1a, 2a zu einer Vernetzung und/oder Verkettung der Molekülketten der ersten Silikonzusammensetzung 1a und der zweiten Silikonzusammensetzung 2a. D.h., es wird im Verbindungsabschnitt 4 auf molekularer Ebene eine Verknüpfung der ersten Silikonzusammensetzung 1a mit der zweiten Silikonzusammensetzung 2a gebildet. Der Aushärtemechanismus, d.h. ob es zu beispielsweise zu einem Vernetzen oder zu einem Verketten und/oder zu beidem kommt, ist dabei von den Eigenschaften der jeweiligen Silikonzusammensetzung abhängig. Die erste Silikonzusammensetzung 1a und die zweite Silikonzusammensetzung 2a werden im Verbindungsabschnitt 4 im Allgemeinen nicht homogen verteilt sein. In der Praxis wird in der Regel die Konzentration der einzelnen Komponenten zu den an den Verbindungsabschnitt 4 anschließenden Abschnitten 1, 2, die die jeweilige Komponente in reiner Form enthalten, höher sein. Insbesondere kann sich die Härte des Verbindungsabschnitts 4 über dessen Ausdehnung kontinuierlich von der Härte des ersten Abschnitts 1 zur Härte des zweiten Abschnitts 2 ändern, wobei die Änderung der Härte nicht unbedingt linear verlaufen muss. Durch das Vernetzen und/oder Verketten der ersten Silikonzusammensetzung la mit der zweiten Silikonzusammensetzung 2a auf molekularer Ebene wird eine dauerhafte und hoch belastbare stoffschlüssige Verbindung des ersten Abschnitts 1 und des zweiten Abschnitts 2 der medizinischen Einrichtung 10 erzielt.

Im Ausführungsbeispiel ist vorgesehen, dass die erste Silikonzusammensetzung 1a im ersten Abschnitt 1 der Einrichtung 10 in reiner Form vorliegt. D.h., dass keine weiteren Materialzusammensetzungen oder Fremdkörper im ersten Abschnitt 1 der medizinischen Einrichtung 10 vorhanden sind. Auch im zweiten Abschnitt 2 ist ausschließlich die zweite Silikonzusammensetzung 2a vorhanden ist. Der Verbindungsabschnitt setzt sich ausschließlich aus einer Mischung der ersten Silikonzusammensetzung 1a und der zweiten Silikonzusammensetzung 2a zusammen. D.h., im Verbindungsabschnitt 4 wird auf weitere Verbindungsmittel, wie z.B. Klebstoffe, oder andere Zuschlagsstoffe verzichtet.

Im vollständig ausgehärteten Zustand der Einrichtung 10, vgl. Fig. 4, weisen die erste und zweite Silikonzusammensetzung 1a, 2a ihre jeweilige Endhärte auf. Aufgrund der Wahl der Silikonzusammensetzungen 1a, 2a ist der erste Abschnitt 1 härter ausgebildet als der zweite Abschnitt 2. D.h., die Einrichtung 10 verfügt über Bereiche unterschiedlicher mechanischer Eigenschaften, die je nach den Erfordernissen der Einrichtung 10 konfiguriert sind.

Bevorzugt weist die erste Silikonzusammensetzung eine Shore-A Härte in einem Bereich von 30 bis 90, und die zweite Silikonzusammensetzung in einem Bereich von 2 bis 15 auf. Diese Wertebereiche der Shore-A Härte sind für ein breites Einsatzspektrum im Bereich der Implantate vorteilhaft. Je nach Erfordernis können aber auch Silikonzusammensetzungen mit davon abweichenden Werten eingesetzt werden.

Die medizinische Einrichtung 10 liegt nach dem Aushärten in einstückiger Form vor.

In den Fig. 5 und 6 ist eine Alternative zu dem in den Fig. 1 bis 3 dargestellten Verfahren gezeigt. Der strukturelle Aufbau der Form 20 entspricht im Wesentlichen jener des ersten Ausführungsbeispiels, sodass in den Erläuterungen zu dieser Alternative hauptsächlich auf die Unterschiede zum in den Fig. 1 bis 3 dargestellten Ausführungsbeispiel hingewiesen wird. Abgesehen von den im Folgenden angeführten Unterschieden gelten die Erläuterungen zum ersten Ausführungsbeispiel des Verfahrens zur Herstellung der medizinischen Einrichtung 10 auch beim zweiten Ausführungsbeispiel.

Im zweiten Ausführungsbeispiel ist vorgesehen, dass die Form 20 einen Trennschieber 26 aufweist, mit dem der Aufnahmeraum 27 in einem Einfüllzustand der Form 20 in zwei Teil-Aufnahmeräume unterteilt werden kann, vgl. Fig. 5. In Fig. 5 ist der Zustand dargestellt, in dem die erste Silikonzusammensetzung 1a und die zweite Silikonzusammensetzung 2a bereits in den Aufnahmeraum 27 eingefüllt sind. Jede der Komponenten ist hierzu in einen der zwei Teil-Aufnahmeräume eingefüllt. Der Trennschieber 26 trennt die beiden Silikonzusammensetzungen 1a, 2a im Einfüllzustand voneinander.

Der verschiebbar gelagerte Trennschieber 26 kann im dargestellten Ausführungsbeispiel vollständig aus dem Bereich des Aufnahmeraums 27 bewegt werden, vgl. Fig. 6. Nach dem Verschieben des Trennschiebers 26 aus dem Bereich des Aufnahmeraums 27 bildet sich der Verbindungsabschnitt 4, in dem sich die erste Silikonzusammensetzung 1a und die zweite Silikonzusammensetzung 2a berühren und vermischen. Das Aushärten der Silikonzusammensetzungen 1a, 2a erfolgt analog zum oben angeführten Beispiel gemäß der Fig. 1 bis 3.

Die mit dem alternativen Verfahren gemäß der Fig. 5 und 6 hergestellte medizinische Einrichtung 10 ist im Wesentlichen identisch zur in Fig. 4 dargestellten Einrichtung der zuvor erläuterten Variante. Durch das Vorsehen eines Trennschiebers 26 kann jedoch die Position des Verbindungsabschnitts 4 der medizinischen Einrichtung 10 genauer definiert werden, ohne dass es dabei nötig ist, ein bestimmtes Volumen einer der beiden Silikonzusammensetzungen 1a, 2a in den Aufnahmeraum 27 zuzuführen, wie dies im ersten Beispiel mittels der Dosierpumpen realisiert ist.

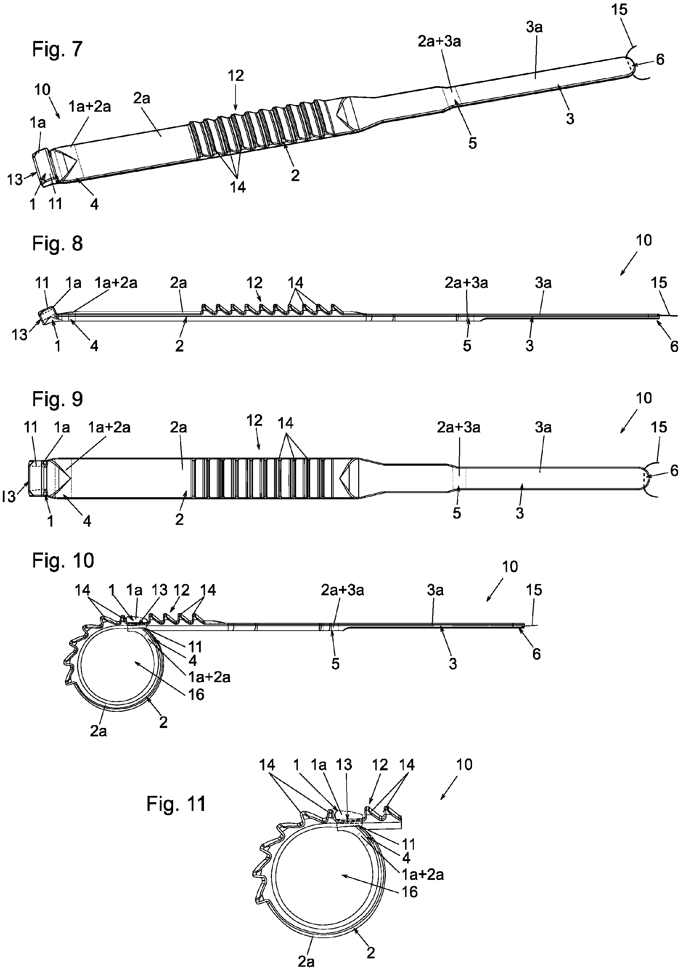

In den Fig. 7 bis 12 ist nun ein konkretes Beispiel einer medizinischen Einrichtung dargestellt, das zum Verengen eines Körperkanals eingesetzt werden kann. Der strukturelle Aufbau der medizinischen Einrichtung hinsichtlich des ersten und zweiten Abschnitts und der jeweiligen Silikonzusammensetzungen sowie des Verbindungsabschnitts entspricht grundsätzlich dem in Fig. 4 gezeigten Ausführungsbeispiel. Im Folgenden wird somit hauptsächlich auf Unterschiede bzw. auf zusätzliche Merkmale im Vergleich zum in Fig. 4 gezeigten Beispiel eingegangen. Insbesondere zur Herstellung der medizinischen Einrichtung wird grundsätzlich auf die Erläuterungen zu den Fig. 1 bis 3 hingewiesen.

Die in den Fig. 7 bis 12 dargestellte medizinische Einrichtung ist zum Verengen eines Körperkanals 30 vorgesehen. Die medizinische Einrichtung 10 weist wiederum einen ersten Abschnitt 1 aus einer ersten Silikonzusammensetzung 1a und einen zweiten Abschnitt 2 aus einer zweiten Silikonzusammensetzung 2a auf, die gegenüber der ersten Silikonzusammensetzung 1a eine geringere Shore-A Härte aufweist. Zwischen dem ersten Abschnitt 1 und dem zweiten Abschnitt 2 ist ein Verbindungsabschnitt 4 angeordnet, der den ersten Abschnitt 1 und den zweiten Abschnitt 2, analog zum Beispiel gemäß Fig. 4, miteinander stoffschlüssig verbindet.

Zusätzlich weist die Einrichtung 10 einen dritten Abschnitt 3 aus einer dritten Silikonzusammensetzung 3a auf. Zwischen dem zweiten Abschnitt 2 und dem dritten Abschnitt 3 ist ein Zusatz-Verbindungsabschnitt 5 angeordnet, der aus einer Mischung der zweiten Silikonzusammensetzung 2a und der dritten Silikonzusammensetzung 3a besteht. In den Figuren ist die Mischung der zweiten und dritten Silikonzusammensetzung 2a, 3a mit 2a+3a bezeichnet.

Um die Abschnitte 1-3 und die Verbindungsabschnitte 4, 5 der Einrichtung 10 in den Fig. 7-10 voneinander optisch unterscheiden zu können, sind diese durch gepunktete Linien voneinander abgegrenzt dargestellt.

Im Ausführungsbeispiel gemäß Fig. 7-12 ist die dritte Silikonzusammensetzung 3a identisch zur ersten Silikonzusammensetzung 1a. Die zweite Silikonzusammensetzung 2a weist somit gegenüber der ersten Silikonzusammensetzung 1a und der dritten Silikonzusammensetzung 3a eine geringere Shore-A Härte auf.

Die zweite Silikonzusammensetzung 2a weist gegenüber der ersten Silikonzusammensetzung 1a eine zumindest um den Wert 30 geringere Shore-A Härte auf. Im Ausführungsbeispiel ist vorgesehen, dass die Härte der ersten Silikonzusammensetzung la 70 Shore-A beträgt. Die dritte Silikonzusammensetzung 3a weist im Ausführungsbeispiel entsprechend auch eine Shore-A Härte von 70 auf.

Die zweite Silikonzusammensetzung 2a weist günstigerweise eine Shore-A Härte in einem Bereich von 2 bis 15 auf. Im Ausführungsbeispiel beträgt die Härte der zweiten Silikonzusammensetzung 2a 5 Shore-A. Silikonzusammensetzungen mit einer Shore-A Härte im genannten Bereich von 2 bis 15 Shore-A sind im Vergleich zu Silikonzusammensetzungen mit einer Shore-A Härte von 40 bis 90 wesentlich dehnbarer.

Die Einrichtung 10 ist bandförmig ausgebildet und um das den Körperkanal 30 umgebende Körpergewebe legbar. Der erste Abschnitt 1 der Einrichtung 10 weist ein erstes Verschlussteil 11 und der zweite Abschnitt 2 ein zweites Verschlussteil 12 auf, mittels denen die Einrichtung 10 zu einem Ring schließbar ist, der eine Durchtrittsöffnung 16 für den Körperkanal 30 umschließt.

Das erste Verschlussteil 11 weist eine Einstecköffnung 13 auf, in die das zweite Verschlussteil 12 einführbar ist. Das zweite Verschlussteil 12 weist eine Vielzahl von Rastnasen 14 auf, die im geschlossenen Zustand der Einrichtung 10 gegenüber dem ersten Verschlussteil 11 zur Einstellung der Größe der Durchtrittsöffnung 16 wahlweise mit dem ersten Verschlussteil 11 verrastbar sind, vgl. Fig. 10 und 11. Aufgrund der geringen Shore-A Härte der zweiten Silikonzusammensetzung 2a kann das zweite Verschlussteil 12 durch eine elastische Verformung der Rastnasen 14 vom Operateur durch die Einführöffnung 13 gezogen werden.

Der dritte Abschnitt 3 der Einrichtung bildet einen bandförmigen Manipulationsabschnitt zum Hindurchführen des zweiten Verschlussteils 12 durch die Einstecköffnung 13. Im Ausführungsbeispiel ist der Querschnitt des Manipulationsabschnitts kleiner als der Querschnitt der Einstecköffnung 13. D.h., der Manipulationsabschnitt ist mit Spiel durch die Einstecköffnung einsteckbar, was das Hindurchfädeln des dritten Abschnitts 3 durch das erste Verschlussteil 11 während der Implantation erleichtert.

Im Ausführungsbeispiel weist die Einrichtung einen zusätzlichen Einführabschnitt 6 auf, in dem ein Manipulationsfaden 15 angeordnet ist. Der Einführabschnitt 6 schließt an den dritten Abschnitt 3 der Einrichtung 10 materialeinstückig an und umfasst einen aus der dritten Silikonzusammensetzung 3a gefertigten Grundkörper, in den der Manipulationsfaden 15 eingegossen ist. Der Manipulationsfaden 15 kann vom Operateur mittels eines entsprechenden Instruments gefasst werden und vereinfacht die Implantation der Einrichtung 10.

Insgesamt ist die medizinische Einrichtung gemäß der Fig. 7-12 einstückig ausgebildet. Abgesehen vom optionalen Manipulationsfaden 15 besteht die medizinische Einrichtung 10 vollständig aus Silikonzusammensetzungen.

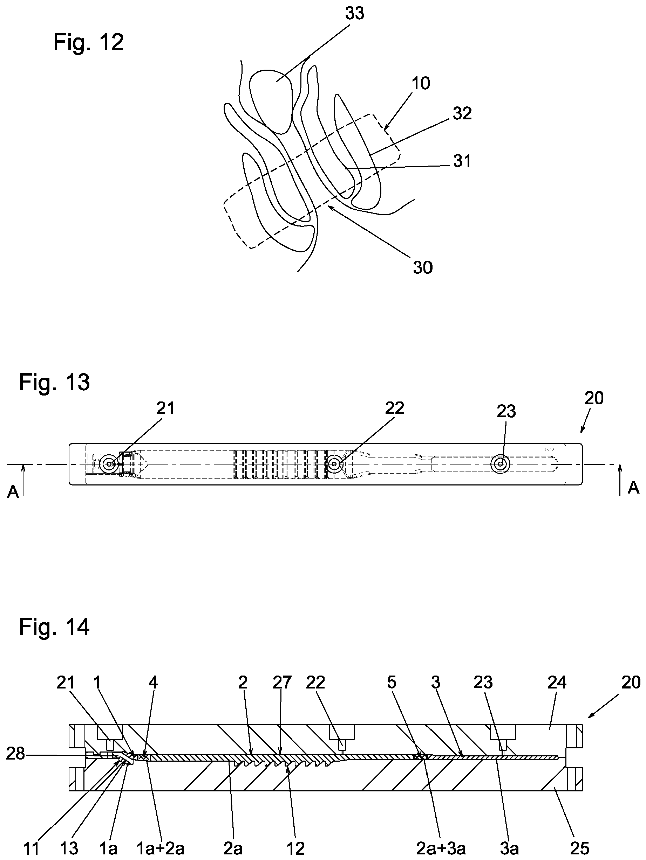

In Fig. 12 ist die Anwendung der Einrichtung 10 als künstlicher Afterschließmuskel im implantierten Zustand schematisch dargestellt, um die Funktion der Einrichtung als künstlicher Sphinkter zu verdeutlichen. Die medizinische Einrichtung 10 wird um den inneren Afterschließmuskel 31 und den äußeren Afterschließmuskel 32 gelegt und unterstützt im geschlossenen Zustand, in dem diese zu einem Ring geschlossen ist, die Verengung des Körperkanals 30, d.h. des Mastdarms. Derartige medizinische Einrichtungen 10 werden, wie eingangs erläutert, auch als Analband zur Behandlung von Stuhlinkontinenz bezeichnet.

Der dritte Abschnitt 3 sowie der Einführabschnitt 6 und für den Zweck der Einrichtung 10 nicht weiter benötigte Bereiche des zweiten Abschnitts 2 können nach dem Schließen des Rings vom Operateur entfernt werden. D.h., die implantierte Einrichtung 10 wird zweckmäßigerweise auf die wesentliche, für die bereitzustellende Funktion benötigte, Länge gekürzt. Im implantierten Zustand weist die medizinische Einrichtung 10 in dieser Anwendung lediglich den ersten Abschnitt 1 und einen Teil des ursprünglichen zweiten Abschnitts 2 sowie den dazwischenliegenden Verbindungsabschnitt 4 auf, vgl. Fig. 11.

Die weiche zweite Silikonzusammensetzung 2a ermöglicht im implantierten Zustand ein elastisches Ausdehnen der medizinischen Einrichtung 10 in radialer Richtung. D.h., zur Defäkation von Stuhl 33 ist die Durchtrittsöffnung 16 der Einrichtung 10 vergrößerbar. Nach dem Ausscheiden des Stuhls 33 aus dem Mastdarm erfolgt eine elastische Rückverformung der Einrichtung 10, die den Verschluss des Mastdarms unterstützt. Insbesondere unterstützt die medizinische Einrichtung 10 die geschwächten Afterschließmuskeln 31, 32.

Wie in Fig. 11 ersichtlich, erstreckt sich der zweite Abschnitt 2 der Einrichtung im Ausführungsbeispiel, im zu einem Ring geschlossenen Zustand der Einrichtung 10, über zumindest 75% des Umfangs der Durchtrittsöffnung 16.

Die härtere Silikonzusammensetzung 1a gewährleistet einen sicheren Verschluss der zu einem Ring geschossenen Einrichtung 10 im implantierten Zustand der Einrichtung. Im Ausführungsbeispiel erstreckt sich der erste Abschnitt 1 über weniger als 20% des Umfangs der Durchtrittsöffnung 16 im zu einem Ring geschlossenen Zustand der Einrichtung 10. Eine geringe Ausdehnung des ersten Abschnitts 1 verbessert den Tragekomfort der Einrichtung 10.

In den Fig. 13 und 14 ist die Form 20 dargestellt, mit der die Herstellung der medizinischen Einrichtung 10 der Fig. 7 bis 12 erfolgen kann. Hinsichtlich des strukturellen Aufbaus weist diese Form 20 zahlreiche Ähnlichkeiten zu dem in den Fig. 1 bis 3 dargestellten schematischen Beispiel auf, sodass in den folgenden Erläuterungen hauptsächlich auf Unterschiede im Vergleich zu der in Fig. 1 bis 3 dargestellten Form 20 eingegangen wird. Abgesehen von den im Folgenden angeführten Unterschieden gelten die Erläuterungen zu den Fig. 1 bis 3 auch für die in Fig. 13 und 14 dargestellte Form 20.

Die in den Fig. 13 und 14 dargestellte Form 20 weist eine obere Formhälfte 24 mit einer ersten Einfüllöffnung 21, einer zweiten Einfüllöffnung 22 und einer dritten Einfüllöffnung 23 auf, die in den Aufnahmeraum 27 münden. Weiters weist die Form 20 ein Einlegeteil 28 auf, das eine Entnahme der medizinischen Einrichtung 10 aus der Form im verfestigten Zustand ermöglicht. Im Weiteren dient das Einlegeteil 28 zur Ausbildung der Einstecköffnung 13 der medizinischen Einrichtung 10, vgl. Fig. 14. Einlegeteile sind aus dem Bereich von Spritzgießwerkzeugen, z.B. zur Herstellung von Hinterschneidungen, grundsätzlich bekannt.

In Fig. 14 ist ein Zustand dargestellt, in dem die erste Silikonzusammensetzung 1a und die zweite Silikonzusammensetzung 2a und die dritte Silikonzusammensetzung 3a im flüssigen Zustand in den Aufnahmeraum 27 eingefüllt sind. Auch der Verbindungsabschnitt 4, in dem die erste Silikonzusammensetzung 1a und die zweite Silikonzusammensetzung 2a vermischt sind, und der Zusatz-Verbindungsabschnitt 5, in dem die zweite Silikonzusammensetzung 2a und die dritte Silikonzusammensetzung 3a miteinander vermischt sind, sind in Fig. 14 dargestellt.

Nach dem Aushärten der medizinischen Einrichtung 10 kann die obere Formhälfte 24 von der unteren Formhälfte 25 abgenommen und das Einlegeteil 28 aus der Einstecköffnung 13 des ersten Verschlussteils 11 der Einrichtung 10 entfernt werden. Gegebenenfalls erfolgt nach dem Entformen der Einrichtung 10 ein Tempern der medizinischen Einrichtung 10.

Die in den Fig. 7-12 gezeigte Einrichtung 10 könnte in einem anderen Anwendungsfall analog als künstlicher Ösophagussphinkter im Bereich der Speiseröhre eingesetzt werden.

Im Anwendungsfall der Einrichtung 10 als Analband, wie es in den Fig. 7-12 gezeigt ist, ist eine elastische Dehnbarkeit der Einrichtung essentiell, weshalb sich der zweite Abschnitt 2 im geschlossenen Zustand über den überwiegenden Teil des Umfangs der Durchtrittsöffnung 16 erstreckt. In anderen Anwendungsfällen der Einrichtung wäre es aber auch denkbar und möglich, dass eine im Wesentlichen starre Einrichtung bereitgestellt werden soll, z.B. als Armierung bzw.

Verstärkung eines Hohlorgans. Dann ist es denkbar und möglich, dass sich die härtere Silikonzusammensetzung über den überwiegenden Teil des Umfangs der Durchtrittsöffnung erstreckt. Beispielsweise könnte das erste Verschlussteil aus einer weniger harten Silikonzusammensetzung als das zweite Verschlussteil gebildet sein, sodass eine gewisse Elastizität bereitgestellt wird, die die Funktion des Verschlussmechanismus verbessert.

In anderen Anwendungsfällen wäre es denkbar und möglich, dass noch weitere Abschnitte unterschiedlicher Silikonzusammensetzung vorgesehen sind. Dies orientiert sich nach den medizinischen Erfordernissen der zu implantierenden Einrichtung. Günstigerweise ist auch bei diesen Varianten vorgesehen, dass zwischen den weiteren Abschnitten aus unterschiedlichen Silikonzusammensetzungen entsprechende Verbindungsabschnitte bzw. Zusatz-Verbindungsabschnitte angeordnet sind, die jeweils aus einer Mischung der in den jeweils angrenzenden Abschnitten eingesetzten Silikonzusammensetzungen bestehen. Auch können gegebenenfalls vorgesehene Verschlussteile an beliebigen der Abschnitte der Einrichtung vorgesehen sein.

### Legende Zu den Hinweisziffern

- 1: erster Abschnitt
- 1a: erste Silikonzusammensetzung
- 2: zweiter Abschnitt
- 2a: zweite Silikonzusammensetzung
- 3: dritter Abschnitt
- 3a: dritte Silikonzusammensetzung
- 4: Verbindungsabschnitt
- 5: Zusatz-Verbindungsabschnitt
- 6: Einführabschnitt
- 10: Einrichtung
- 11: erstes Verschlussteil
- 12: zweites Verschlussteil
- 13: Einstecköffnung
- 14: Rastnase
- 15: Manipulationsfaden
- 16: Durchtrittsöffnung
- 20: Form
- 21: erste Einfüllöffnung
- 22: zweite Einfüllöffnung
- 23: dritte Einfüllöffnung
- 24: obere Formhälfte
- 25: untere Formhälfte
- 26: Trennschieber
- 27: Aufnahmeraum
- 28: Einlegeteil
- 30: Körperkanal
- 31: innerer Afterschließmuskel
- 32: äußerer Afterschließmuskel
- 33: Stuhl

## Patentansprüche

1. Bandförmige medizinische Einrichtung (10) zum Verengen eines Körperkanals (30) eines Patienten, wobei die Einrichtung (10) um das den Körperkanal (30) umgebende Körpergewebe legbar ist, und wobei die Einrichtung (10) umfasst:
- einen ersten Abschnitt (1) aus einer ersten Silikonzusammensetzung (1a), und
- einen zweiten Abschnitt (2) aus einer zweiten Silikonzusammensetzung (2a), die gegenüber der ersten Silikonzusammensetzung (1a) eine geringere Shore-A Härte aufweist, und
- einen Verbindungsabschnitt (4), der den ersten Abschnitt (1) mit dem zweiten Abschnitt (2) stoffschlüssig verbindet,
wobei der erste Abschnitt (1) ein erstes Verschlussteil (11) und der zweite Abschnitt (2) ein zweites Verschlussteil (12) aufweist, mittels denen die Einrichtung (10) zu einem Ring schließbar ist, der eine Durchtrittsöffnung (16) für das den Körperkanal (30) umgebende Körpergewebe umschließt,
**dadurch gekennzeichnet, dass** der erste und zweite Abschnitt (1, 2), in einer Längserstreckungsrichtung der bandförmigen Einrichtung gesehen, hintereinander angeordnet sind und dass der Verbindungsabschnitt (4) aus einer Mischung der ersten Silikonzusammensetzung (1a) und der zweiten Silikonzusammensetzung (2a) besteht.

2. Einrichtung (10) nach Anspruch 1, **dadurch gekennzeichnet, dass** die zweite Silikonzusammensetzung (2a) eine gegenüber der ersten Silikonzusammensetzung (1a) zumindest um den Wert 15, vorzugsweise zumindest um den Wert 30, geringere Shore-A Härte aufweist.

3. Einrichtung (10) nach Anspruch 1 oder 2, **dadurch gekennzeichnet, dass** die erste Silikonzusammensetzung (1a) eine Shore-A Härte in einem Bereich von 30 bis 70, vorzugsweise in einem Bereich von 40 bis 60, aufweist, und die zweite Silikonzusammensetzung (2a) eine Shore-A Härte in einem Bereich von 2 bis 15, vorzugsweise in einem Bereich von 3 bis 10, besonders bevorzugt in einem Bereich von 4 bis 7, aufweist.

4. Einrichtung (10) nach einem der Ansprüche 1 bis 3, **dadurch gekennzeichnet, dass** das erste Verschlussteil (11) eine Einstecköffnung (13) aufweist.

5. Einrichtung (10) nach Anspruch 4, **dadurch gekennzeichnet, dass** das zweite Verschlussteil (12) in die Einstecköffnung (13) einführbar ist und gegenüber dem ersten Verschlussteil (11) im geschlossenen Zustand verrastbar ist.

6. Einrichtung (10) nach Anspruch 5, **dadurch gekennzeichnet, dass** das zweite Verschlussteil (12) zumindest zwei Rastnasen (14) aufweist, die zur Einstellung der Größe der Durchtrittsöffnung (16) wahlweise mit dem ersten Verschlussteil (11) verrastbar sind.

7. Einrichtung (10) nach einem der Ansprüche 1 bis 6, **dadurch gekennzeichnet, dass** die Einrichtung (10) einen dritten Abschnitt (3) aus einer dritten Silikonzusammensetzung (3a), wobei der erste, der zweite und der dritte Abschnitt (1, 2, 3), in einer Längserstreckungsrichtung der bandförmigen Einrichtung gesehen, hintereinander angeordnet sind, und einen Zusatz-Verbindungsabschnitt (5) umfasst, der den dritten Abschnitt (3) mit dem zweiten Abschnitt (2) stoffschlüssig verbindet, wobei der Zusatz-Verbindungsabschnitt (5) aus einer Mischung der zweiten Silikonzusammensetzung (2a) und der dritten Silikonzusammensetzung (3a) besteht.

8. Einrichtung (10) nach Anspruch 7 und einem der Ansprüche 4 bis 6, **dadurch gekennzeichnet, dass** der dritte Abschnitt (3) einen bandförmigen Manipulationsabschnitt zum Hindurchführen des zweiten Verschlussteils (12) durch die Einstecköffnung (13) bildet.

9. Einrichtung (10) nach Anspruch 7 oder 8, **dadurch gekennzeichnet, dass** die dritte Silikonzusammensetzung (3a) eine Shore-A Härte in einem Bereich von 30 bis 70, vorzugsweise in einem Bereich von 40 bis 60, aufweist.

10. Verfahren zur Herstellung einer medizinischen Einrichtung (10) nach einem der Ansprüche 1 bis 9, **dadurch gekennzeichnet, dass** die erste Silikonzusammensetzung (1a) in einem flüssigen Zustand über eine im Bereich des auszubildenden ersten Abschnitts (1) mündende erste Einfüllöffnung (21) in einen Aufnahmeraum (27) einer Form (20) eingefüllt wird und die zweite Silikonzusammensetzung (2a) in einem flüssigen Zustand über eine im Bereich des auszubildenden zweiten Abschnitts (2) mündende zweite Einfüllöffnung (22) in den Aufnahmeraum (27) der Form (20) eingefüllt wird, wobei sich die erste Silikonzusammensetzung (1a) und die zweite Silikonzusammensetzung (2a) im flüssigen Zustand im Aufnahmeraum (27) berühren und sich über den Bereich des auszubildenden Verbindungsabschnitts (4) vermischen, wobei zur Bildung der medizinischen Einrichtung (10) die erste Silikonzusammensetzung (1a) im ersten Abschnitt (1) der Einrichtung (10) und die zweite Silikonzusammensetzung (2a) im zweiten Abschnitt (2) der Einrichtung (10) und die Mischung der ersten Silikonzusammensetzung (1a) und der zweiten Silikonzusammensetzung (2a) im Verbindungsabschnitt (4) ausgehärtet werden.

## Claims

1. A band-shaped medical device (10) for narrowing a body channel (30) of a patient, the device (10) being placeable around the body tissue surrounding the body channel (30), and the device (10) comprising:
- a first section (1) composed of a first silicone composition (1a), and
- a second section (2) composed of a second silicone composition (2a) having a lower Shore A hardness compared to the first silicone composition (1a), and
- a connecting section (4) which integrally bonds the first section (1) to the second section (2),
wherein the first section (1) has a first locking part (11) and the second section (2) has a second locking part (12), by means of which the device (10) is closable to form a ring which encircles a passage opening (16) for the body tissue surrounding the body channel (30), **characterized in that** the first and second section are arranged one after the other, viewed in a direction of longitudinal extent of the band-shaped device, and that the connecting section (4) consists of a mixture of the first silicone composition (1a) and the second silicone composition (2a).

2. The device (10) as claimed in claim 1, **characterized in that** the second silicone composition (2a) has a Shore A hardness lower at least by the value of 15, preferably at least by the value of 30, compared to the first silicone composition (1a).

3. The device (10) as claimed in claim 1 or 2, **characterized in that** the first silicone composition (1a) has a Shore A hardness in a range from 30 to 70, preferably in a range from 40 to 60, and the second silicone composition (2a) has a Shore A hardness in a range from 2 to 15, preferably in a range from 3 to 10, particularly preferably in a range from 4 to 7.

4. The device (10) as claimed in any of claims 1 to 3, **characterized in that** the first locking part (11) has an insertion opening (13).

5. The device (10) as claimed in claim 4, **characterized in that** the second locking part (12) is insertable into the insertion opening (13) and is latchable with respect to the first locking part (11) in the closed state.

6. The device (10) as claimed in claim 5, **characterized in that** the second locking part (12) has at least two latching lugs (14) which are latchable with the first locking part (11) as desired to adjust the size of the passage opening (16).

7. The device (10) as claimed in any of claims 1 to 6, **characterized in that** the device (10) comprises a third section (3) composed of a third silicone composition (3a), wherein the first, second and third section are arranged one after the other, viewed in a direction of longitudinal extent of the band-shaped device, and an additional connecting section (5) which integrally bonds the third section (3) to the second section (2), the additional connecting section (5) consisting of a mixture of the second silicone composition (2a) and the third silicone composition (3a).

8. The device (10) as claimed in claim 7 and any of claims 4 to 6, **characterized in that** the third section (3) forms a band-shaped manipulation section for passing the second locking part (12) through the insertion opening (13).

9. The device (10) as claimed in claim 7 or 8, **characterized in that** the third silicone composition (3a) has a Shore A hardness in a range from 30 to 70, preferably in a range from 40 to 60.

10. A method for producing a medical device (10) as claimed in any of claims 1 to 9, **characterized in that** the first silicone composition (1a) is filled in a liquid state into an accommodation space (27) of a mold (20) via a first fill opening (21) leading to the region of the first section (1) to be formed and the second silicone composition (2a) is filled in a liquid state into the accommodation space (27) of the mold (20) via a second fill opening (22) leading to the region of the second section (2) to be formed, wherein the first silicone composition (1a) and the second silicone composition (2a) touch one another in the accommodation space (27) in the liquid state and mixing with one another over the region of the connecting section (4) to be formed, wherein for forming the medical device (10) the first silicone composition (1a) is cured in the first section (1) of the device (10) and the second silicone composition (2a) is cured in the second section (2) of the device (10) and the mixture of the first silicone composition (1a) and the second silicone composition (2a) is cured in the connecting section (4).

## Revendications

1. Dispositif médical en forme de bande (10) destiné à rétrécir un canal corporel (30) d'un patient, le dispositif (10) pouvant être disposé autour du tissu corporel entourant le canal corporel (30), et le dispositif (10) comprenant :
- une première section (1) faite d'une première composition de silicone (1a), et
- une deuxième section (2) faite d'une deuxième composition de silicone (2a), ayant une dureté Shore-A inférieure à celle de la première composition de silicone (1a), et
- une section de raccordement (4) qui relie par continuité de matière la première section (1) à la deuxième section (2),
le premier segment (1) comprenant une première partie de verrouillage (11) et la deuxième section (2) comprenant une deuxième partie de verrouillage (12), au moyen desquelles le dispositif (10) peut être fermé en formant un anneau qui entoure une ouverture de passage (16) pour le tissu corporel entourant le canal corporel (30), **caractérisé en ce que** la première et la deuxième section (1, 2), considérées dans une direction longitudinale du dispositif en forme de bande, sont disposées l'une derrière l'autre, et **en ce que** la section de raccordement (4) est composée d'un mélange de la première composition de silicone (1a) et de la deuxième composition de silicone (2a).

2. Dispositif (10) selon la revendication 1, **caractérisé en ce que** la deuxième composition de silicone (2a) présente, par rapport à la première composition de silicone (1a), une dureté Shore-A inférieure d'au moins 15, de préférence d'au moins 30.

3. Dispositif (10) selon la revendication 1 ou 2, **caractérisé en ce que** la première composition de silicone (1a) présente une dureté Shore-A dans la plage comprise entre 30 et 70, de préférence dans la plage comprise entre 40 et 60, et la deuxième composition de silicone (2a) présente une dureté Shore-A dans la plage comprise entre 2 et 15, de préférence dans la plage comprise entre 3 et 10, et particulièrement préférée dans la plage comprise entre 4 et 7.

4. Dispositif (10) selon l'une des revendications 1 à 3, **caractérisé en ce que** la première partie de verrouillage (11) comprend une ouverture d'insertion (13).

5. Dispositif (10) selon la revendication 4, **caractérisé en ce que** la deuxième partie de verrouillage (12) peut être insérée dans l'ouverture d'insertion (13) et peut être encliquetée, à l'état fermé, par rapport à la première partie de verrouillage (11).

6. Dispositif (10) selon la revendication 5, **caractérisé en ce que** la deuxième partie de verrouillage (12) comprend au moins deux crochets de verrouillage (14) qui peuvent être encliquetés au choix avec la première pièce de fermeture (11) pour régler la taille de l'ouverture de passage (16).

7. Dispositif (10) selon l'une des revendications 1 à 6, **caractérisé en ce que** le dispositif (10) comprend une troisième section (3) faite d'une troisième composition de silicone (3a), les première, deuxième et troisième sections (1, 2, 3) étant, considérées dans une direction longitudinale du dispositif en forme de bande, disposées l'une derrière l'autre, ainsi qu'une section de raccordement additionnelle (5) qui relie par continuité de matière la troisième section (3) à la deuxième section (2), la section de raccordement additionnelle (5) étant constituée d'un mélange de la deuxième composition de silicone (2a) et de la troisième composition de silicone (3a).

8. Dispositif (10) selon la revendication 7 et l'une des revendications 4 à 6, **caractérisé en ce que** la troisième section (3) forme une section de manipulation en forme de bande permettant de faire passer la deuxième partie de verrouillage (12) à travers l'ouverture d'insertion (13).

9. Dispositif (10) selon la revendication 7 ou 8, **caractérisé en ce que** la troisième composition de silicone (3a) présente une dureté Shore-A dans la plage comprise entre 30 et 70, de préférence dans la plage comprise entre 40 et 60.

10. Procédé de fabrication d'un dispositif médical (10) selon l'une des revendications 1 à 9, **caractérisé en ce que** la première composition de silicone (1a) est introduite dans un état liquide dans une chambre de réception (27) par une première ouverture de remplissage (21) débouchant au niveau de la première section (1) à former, et la deuxième composition de silicone (2a) est introduite dans un état liquide dans la chambre de réception (27) par une deuxième ouverture de remplissage (22) débouchant au niveau de la deuxième section (2) à former, la première composition de silicone (1a) et la deuxième composition de silicone (2a) étant en contact dans la chambre de réception (27) à l'état liquide et se mélangeant dans la zone de la section de raccordement (4), la première composition de silicone (1a) durcissant dans la première section (1) du dispositif et la deuxième composition de silicone (2a) durcissant dans la deuxième section (2) du dispositif, et le mélange de la première composition de silicone (1a) et de la deuxième composition de silicone (2a) durcissant dans la section de raccordement (4) pour former le dispositif médical (10).
